# DEMANDE DE BREVET EUROPEEN

(11) **EP 2 886 535 A1**
(43) Date de publication de la demande: **24.06.2015**
(21) Numéro de dépôt: 13306841.1
(22) Date de dépôt: 23.12.2013
(51) Int. Cl.: C07D 221/12, A61K 9/00, A61K 47/02, A61K 9/08, A61K 31/473, A61P 33/02

(54) **Nouvelles compositions vétérinaires à base d'isométamidium et substances apparentées et leurs utilisations**

(71) Demandeur: Ceva Sante Animale, 33500 Libourne Cedex (FR)
(72) Inventeur: Karembe, Hamadi, 33500 LIBOURNE (FR)
(74) Mandataire: Becker, Philippe

(57) **Abrégé**

La présente invention est relative à des compositions vétérinaires à base d'isométamidium et leurs utilisations dans un traitement curatif et/ou préventif à long terme des trypanosomoses chez les mammifères non-humains. Elle concerne plus spécifiquement une composition comprenant de l'isométamidium pur à au moins 95%, ou un de ses sels, par rapport au poids total de la composition. Les compositions de l'invention comprennent en outre, du 3,8-di-(3-*m*-amidinophenyltriazeno)-5-éthyl-6-phénylphénanthridium pur et, optionnellement, moins de 8 % en poids de 3-(3-*m-*amidinophényl-2-triazeno)-8-amino-5-éthyl-6-phénylphénanthridinium et moins de 8 % en poids de 7-(*m*-amidinophenyldiazo)-3,8-diamino-5-ethyl-6-phenylphenanthridinium.

## Description

La présente invention est relative à de nouvelles compositions vétérinaires à base d'isométamidium ou un de ses sels, ainsi qu'à leurs utilisations. L'invention concerne plus spécifiquement de nouvelles compositions vétérinaires comprenant de l'isométamidium pur à au moins 95 %. L'invention concerne également des compositions vétérinaires d'isométamidium pur comprenant d'autres actifs, dans des quantités contrôlées et optimisées pour une meilleure efficacité, et en particulier le composé disubstitué. Les compositions vétérinaires de l'invention peuvent être utilisées notamment dans le traitement des trypanosomoses chez les mammifères non-humains.

### ARRIERE-PLAN TECHNOLOGIQUE DE L'INVENTION

Les trypanosomoses sont des maladies parasitaires provoquées par des protozoaires inoculés par des vecteurs. La trypanosomose animale sévit dans les zones tropicales et intertropicales d'Afrique, d'Asie et d'Amérique. En l'absence de traitements trypanocides, les animaux trypanosensibles meurent tandis que les animaux dits trypanotolérants connaissent une baisse considérable de leur productivité et un taux de mortalité élevé au vêlage.

La trypanosomose africaine (T.A), transmise par les glossines, est une maladie parasitaire endémique touchant de nombreux pays et est causée par *Trypanosoma brucei brucei* (*T. b. brucei*), *Trypanosoma congolense* (*T. congolense*) et *Trypanosoma vivax* (*T. vivax*). Cette maladie est présente sur environ un tiers des superficies terrestres de l'Afrique et a des effets considérables sur l'agriculture. On estime que les pertes annuelles de production bovine à elles seules sont de l'ordre de 1 à 1,5 milliard de dollars. Les bovins, dans cette région, ne peuvent être utilisés comme animaux de trait, et leur «valeur nutritionnelle» est faible. Elle affecte également les porcs, les chameaux, les chèvres et les moutons. Plus de 30 espèces de mammifères non humains sauvages ou domestiques comme les chiens, chats et singes sont également susceptibles à cette infection et peuvent servir de réservoirs pour les trypanosomes pathogènes pour l'homme. L'importance des enjeux de travaux visant à lutter contre cette maladie a été signalée par le programme mondial pour la lutte contre les maladies négligées (DNDi/OMS) comme une priorité humanitaire au niveau mondial.

Les produits disponibles pour traiter la trypanosomose animale sont principalement le diminazène (Veriben® - Ceva Santé Animale, Berenil® - Merck), l'isométamidium (Veridium® - Ceva Santé Animale, Samorin® - Mérial) et l'homidium (Novidium® - Mérial). Ces produits n'ont que peu évolué depuis 50 ans. En outre leur mode d'action et/ou composition sont parfois mal définis et donc mal contrôlés. Ainsi, le composé désigné isométamidium est en fait un mélange complexe de plusieurs substances, dont la nature et la quantité peuvent varier en fonction du procédé d'obtention. Les principales voies de fabrication de l'isométamidium ont été développées dans les années 50 à partir de l'homidium et conduisent à l'obtention d'un mélange de différents composés comprenant notamment le 8-(3-m-amidinophényl-2-triazeno)-3-amino-5-éthyl-6-phényl-phénanthridinium (isométamidium - M&B4180), le 3-(3-m-amidinophényl-2-triazeno)-8-amino-5-éthyl-6-phénylphénanthridinium (isomère rouge - M&B3989), le 7-(*m*-amidinophényldiazo)-3,8-diamino-5-éthyl-6-phénylphénanthridinium (isomère bleu - M&B4250) et le 3,8-di-(3-m-amidinophenyltriazeno)-5-éthyl-6-phénylphénanthridium (composé jaune ou disubstitué - M&B4596).

Devant la complexité, l'aspect peu pratique, les coûts des procédés de purification et les premières données biologiques analysées, les mélanges ont ainsi été commercialisés tels quels et vendus sous le nom générique d'isométamidium. On peut citer par exemple les formulations commercialisées sous les dénominations Veridium® de Ceva Santé Animale et Samorin® de Merial, qui correspondent à des mélanges complexes de différentes substances apparentées à l'isométamidium. Ainsi, la formulation Veridium® comprend 70 à 80 % de 8-(3-m-amidinophényl-2-triazeno)-3-amino-5-éthyl-6-phénylphénanthridinium (isométamidium), 8 à 15 % d'isomère rouge, 8 à 15 % d'isomère bleu, près de 4 % du composé disubstitué, et environ moins de 1 % d'homidium. La formulation Samorin® comprend 55 à 65 % de 8-(3-m-amidinophényl-2-triazeno)-3-amino-5-éthyl-6-phényl-phénanthridinium (isométamidium), 10 à 20 % d'isomère rouge, 10 à 20 % d'isomère bleu, 5 à 10 % du composé disubstitué et environ moins de 1 % d'homidium.

Dès les années 60, des travaux scientifiques ont essayé d'évaluer l'activité trypanocide de certains composés constituant l'isométamidium. Les résultats de ces travaux demeurent néanmoins incomplets et incertains.

Ainsi, Brown et al. (Br. J. Pharmacol. Chemother., 1961, 17, 396-405) ont évalué l'activité trypanocide *in-vivo* sur des souris de composés présentées comme étant l'isomère bleu (M&B4250), l'isométamidium (M&B4180), le composé disubstitué (M&B4596) et un mélange du composé rouge et bleu (métamidium ou M&B4404). Sur la base de leur expérimentation, ils ont conclu à une activité trypanocide curative contre *T. congolense* pour le 7-(*m*-amidinophényldiazo)-3,8-diamino-5-éthyl-6-phénylphénanthridinium (isomère bleu - M&B4250) et une activité prophylactique et non curative du composé disubstitué 3,8-di-(3-*m*-amidinophenyltriazeno)-5-éthyl-6-phénylphénanthridium (M&B4596). Cependant, les auteurs soulignent que les méthodes de synthèse n'ont pas permis d'obtenir des composés avec des niveaux de pureté satisfaisants. Finelle et al. (Rev.Elev.Med.Vet. Pays trop.1963, 16, 413-415) a suggéré que le composé 3,8-di-(3-*m*-amidinophenyltriazeno)-5-éthyl-6-phénylphénanthridium (M&B4596) permettait de protéger les bovins contre *T. congolense,* mais était dépourvu de toute activité curative.

Les difficultés d'analyse des actifs utilisés dans ces études rendent incertaines leurs conclusions ainsi que toutes les données pharmacologiques recueillies depuis des années. Ainsi, à ce jour, aucune étude n'a montré spécifiquement et définitivement l'activité ou les propriétés de l'isométamidium pur et il existe un besoin pour des compositions définies et contrôlée ayant une activité trypanocide à large spectre.

### RESUME DE L'INVENTION

La présente demande fournit de nouvelles compositions vétérinaires à base d'isométamidium pur, ayant une composition définie, contrôlée et optimisée sur le plan biologique, et leurs utilisations pour le traitement curatif et/ou préventif des trypanosomoses chez les mammifères non-humains.

La demanderesse a synthétisé et purifié, avec un degré de pureté supérieur à 95%, le 8-(3-m-amidinophényl-2-triazeno)-3-amino-5-éthyl-6-phénylphénanthridinium (Isométamidium).

La demanderesse a montré, de manière surprenante, que des compositions comprenant de l'isométamidium pur sont plus efficaces lorsqu'elles sont utilisées dans des traitements curatifs des trypanosomoses, par rapport aux formulations d'isométamidium de référence : le Veridium^{®} et le Samorin^{®} ; et que cet effet pouvait être modulé par la présence contrôlée des isomères bleu (M&B4250) et/ou rouge (M&B3989). De plus, la demanderesse a montré, de manière surprenante, que l'addition du composé disubstitué (M&B4596) pur à une composition d'isométamidium pur permettait de maintenir une activité trypanocide efficace à long terme.

L'objet de la présente invention concerne donc une composition vétérinaire comprenant de l'isométamidium pur à au moins 95 % ou un de ses sels et un excipient acceptable en vétérinaire. Dans un mode préféré, la composition vétérinaire comprend au moins 55 %, 80 %, 85 % et, encore plus préférentiellement, au moins 90 % en poids d'isométamidium pur ou un de ses sels.

Un autre objet de l'invention concerne une composition vétérinaire comprenant de l'isométamidium pur à au moins 95 % ou un de ses sels, du 3,8-di-(3-m-amidinophényltriazeno)-5-éthyl-6-phénylphénanthridium pur ou un de ses sels, et un excipient acceptable en vétérinaire.

De préférence, les compositions vétérinaires de l'invention comprennent moins de 8% en poids de 3-(3-*m*-amidinophényl-2-triazeno)-8-amino-5-éthyl-6-phénylphénanthridinium ou de 7-(*m*-amidinophényldiazo)-3,8-diamino-5-éthyl-6-phénylphénanthridinium, plus préférentiellement moins de 5%, encore plus préférentiellement, elles sont dépourvues de ces composés.

Un autre objet de l'invention concerne les compositions vétérinaires telles que définies ci-dessus pour leur utilisation dans le traitement des trypanosomoses et/ou contre les trypanosomes chez les mammifères non-humains, en particulier pour le traitement curatif et/ou préventif des trypanosomoses chez les mammifères non-humains dues par exemple aux trypanosomes *T. congolense*, *T. b. brucei*, *T.brucei*, *T. evansi* et *T. vivax.*

L'invention concerne également une méthode de traitement d'une maladie parasitaire causée par un trypanosome chez un mammifère non-humain, comprenant l'administration au dit mammifère non-humain d'une composition vétérinaire telle que définie ci-dessus. Le traitement peut être curatif et/ou préventif, et à long terme.

L'invention concerne aussi une méthode ou un procédé de préparation d'une composition vétérinaire telle que définie ci-dessus, comprenant les étapes consistant à préparer et/ou purifier séparément les différents ingrédients, et à les assembler ensuite.

Les compositions de l'invention sont utilisables chez les mammifères non-humains tels que notamment les bovidés, les ovidés, les camélidés, les équidés ou les canidés. Elles peuvent être administrées par exemple par voie parentérale, de préférence par injection intramusculaire ou sous-cutanée.

L'invention concerne aussi le chlorure d'isométamidium pur à au moins 95 %, ou un de ses sels, en particulier le chlorure de chlorhydrate d'isométamidium. L'invention concerne encore le chlorure de 3,8-di-(3-*m*-amidinophényltriazeno)-5-éthyl-6-phénylphénanthridium pur à au moins 95 % ou un de ses sels, en particulier le chlorure de dichlorhydrate 3,8-di(3-m-amidinophényltriazeno)-5-éthyl-6-phénylphénanthridium.

### LEGENDE DES FIGURES

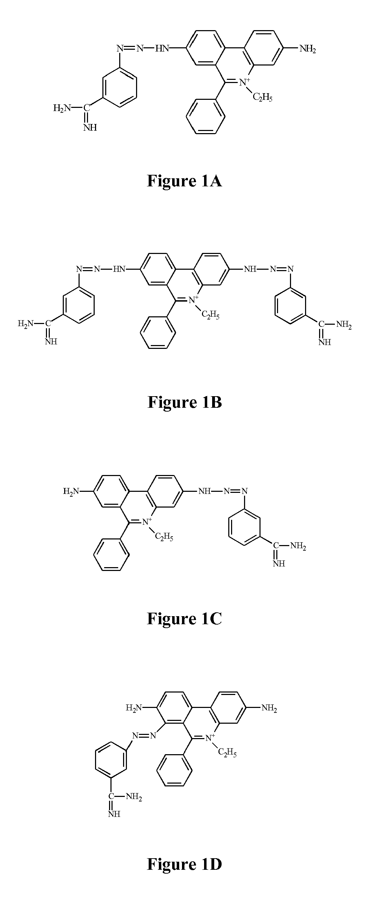

- **Figure 1A** : Structure chimique de l'isométamidium ou 8-(3-m-amidinophényl-2-triazeno)-3-amino-5-éthyl-6-phényl-phénanthridinium (M&B 4180)
- **Figure 1B** : Structure chimique du 3,8-di-(3-*m*-amidinophenyltriazeno)-5-éthyl-6-phénylphénanthridium ou composé disubstitué (M&B4596)
- **Figure 1C** : Structure chimique du 3-(3-*m*-amidinophényl-2-triazeno)-8-amino-5-éthyl-6-phényl phénanthridinium (isomère rouge - M&B3989)
- **Figure 1D** : Structure chimique du 7-(*m*-amidinophényldiazo)-3,8-diamino-5-éthyl-6-phényl phénanthridinium (isomère bleu - M&B4250)

### DESCRIPTION DETAILLEE DE L'INVENTION

La présente invention est relative à de nouvelles compositions vétérinaires à base d'isométamidium pur et à leurs utilisations dans un traitement curatif et/ou préventif, en particulier à long terme, des trypanosomoses chez les mammifères non-humains.

### Isométamidium et composés

Dans le contexte de la présente invention, on entend par composé « isométamidium », le composé 8-(3-m-amidinophényl-2-triazeno)-3-amino-5-éthyl-6-phénylphénanthridinium (M&B 4180), dont la formule est illustrée dans la figure 1A, ainsi que tout sel de celui-ci. L'isométamidium est une phénantridine cationique.

On entend par « composé disubstitué », le 3,8-di-(3-m-amidinophenyltriazeno)-5-éthyl-6-phénylphénanthridium (M&B4596), dont la formule est illustrée dans la figure 1B, ainsi que tout sel de celui-ci.

Par « isomère rouge», on désigne le 3-(3-m-amidinophényl-2-triazeno)-8-amino-5-éthyl-6-phénylphénanthridinium (M&B3989) répondant à la formule illustrée dans la figure 1C, et par « isomère bleu», on entend le7-(*m*-amidinophényldiazo)-3,8-diamino-5-éthyl-6-phényl phénanthridinium (M&B4250), répondant à la formule illustrée dans la figure 1D.

Le terme « pur » désigne au sens de l'invention un produit ou composé essentiellement dépourvu d'autres composés, contaminants et impuretés, tels que des produits secondaires ou des produits de dégradation pouvant se former lors de leur synthèse chimique. De préférence, le terme « pur » désigne un composé comprenant moins de 5 % d'autres espèces moléculaires, d'impuretés et contaminants, plus particulièrement moins de 4 %, moins de 3 %, moins de 2%, et encore plus préférentiellement moins de 1 % d'autres espèces moléculaires, d'impuretés et de contaminants.

Plus particulièrement, le terme « isométamidium pur » désigne le composé 8-(3-m-amidinophényl-2-triazeno)-3-amino-5-éthyl-6-phénylphénanthridinium, ainsi que tout sel de celui-ci, comprenant moins de 5% d'autres composés, d'impuretés ou contaminants, plus particulièrement moins de 5% d'isomère rouge, d'isomère bleu et de composé jaune. Plus préférentiellement, le terme « isométamidium pur » désigne le composé 8-(3-*m*-amidinophényl-2-triazeno)-3-amino-5-éthyl-6-phénylphénanthridinium, ainsi que tout sel de celui-ci, comprenant moins de 4%, de préférence moins de 3%, encore plus préférentiellement moins de 2%, tout préférentiellement moins de 1% d'isomère rouge, d'isomère bleu et de composé jaune. Un composé isométamidium pur préféré au sens de l'invention est pur à plus de 99%, notamment à au moins 99.7%.

Plus particulièrement, le terme « composé disubstitué pur » désigne le composé 3,8-di-(3-m-amidinophényltriazeno)-5-éthyl-6-phénylphénanthridium, ainsi que tout sel de celui-ci, comprenant moins de 5% d'autres composés, d'impuretés ou contaminants, plus particulièrement moins de 5% d'isométamidium, d'isomère rouge et d'isomère bleu. Plus préférentiellement, le terme « composé disubstitué pur » désigne le composé 3,8-di-(3-m-amidinophenyltriazeno)-5-éthyl-6-phénylphénanthridium, ainsi que tout sel de celui-ci, comprenant moins de 4%, de préférence moins de 3%, d'isométamidium, d'isomère rouge et d'isomère bleu. Un composé disubstitué pur au sens de l'invention est pur à plus de 97%, notamment à au moins 97,9%.

Le degré de pureté peut être évalué par toute méthode analytique connue de l'homme du métier, telle que par exemple par HPLC (Chromatographie Haute Performance Liquide), chromatographie en phase gazeuse (CPG), spectrométrie de masse, ou résonnance magnétique nucléaire. Ces méthodes analytiques peuvent, bien entendu, être couplées ou combinées entre elles.

Les composés selon l'invention peuvent être synthétisés par des méthodes connues en soi. Ainsi, ils peuvent être produits par synthèse selon la méthode de couplage entre l'éthidium et le p-aminophényldiazonium décrite par Wrag et al. 1958, Nature, 182, 1005-1006 ; et par Berg et al. 1960, Nature, 188, 1106, puis purifiés par des techniques mentionnées ci-dessus. Le composé peut également être synthétisé selon des méthodes de synthèse optimisées sans purification.

Dans le contexte de la présente invention, les sels des composés sont par exemple des chlorhydrates, des bromhydrates, des acéturates, des méthanes sulfonates, et tous les autres sels acceptables connus de l'homme du métier. Les sels des composés de l'invention sont préférentiellement des chlorhydrates ou du chlorure de chlorhydrates.

### Compositions vétérinaire d'isométamidium pur

La présente demande concerne toute composition vétérinaire à base d'isométamidium pur, de préférence à base d'isométamidium pur à au moins 95%. Les compositions vétérinaires selon l'invention présentent avantageusement une composition définie, assurant un meilleur contrôle de l'activité trypanocide.

Typiquement, les compositions vétérinaires de l'invention comprennent une quantité d'au moins 55 % en poids d'isométamidium pur ou d'un de ses sels, et un excipient vétérinairement acceptable. Dans des modes de mise en oeuvre particuliers, les compositions de l'invention comprennent entre 55 et 99% en poids d'isométamidium pur ou d'un de ses sels, par exemple au moins 65%, 70%, 75%, 80%, 85%, 90% ou 95% en poids d'isométamidium pur ou d'un de ses sels.

Dans la présente demande, les proportions en poids des composés sont définies par rapport au poids total de la composition les comprenant.

Comme indiqué dans les exemples, les inventeurs ont montré de manière surprenante que l'isométamidium purifié possédait une activité supérieure aux produits commerciaux de référence. En particulier, l'isométamidium pur présente de manière étonnante une activité trypanocide 3 à 4 fois supérieure par rapport aux formulations commerciales, notamment par rapport à la formulation Samorin^{®} sur la souche *T*. *congolense*.

Par ailleurs, les inventeurs ont montré de manière surprenante que l'association d'isométamidium pur et de composé disubstitué pur permettait d'obtenir des compositions vétérinaires ayant des propriétés trypanocides particulièrement avantageuses pour un traitement à long terme. Ainsi, comme montré dans l'exemple 3, l'isométamidium pur et le composé disubstitué pur, lorsqu'ils sont injectés à une dose de 1 mg/kg, permettent d'obtenir une activité curative et préventive pendant au moins trois mois alors que l'activité trypanocide observée pour l'isomère bleu n'est plus significative au bout de deux mois, et nulle pour l'isomère rouge au bout de trois mois.

De ce fait, dans un mode particulier, les compositions de l'invention comprennent en outre du composé disubstitué pur ou un de ses sels, de préférence au moins 4 % en poids du composé disubstitué pur ou un de ses sels. Avantageusement, la composition comprend entre 4 et 30% en poids du composé disubstitué pur ou un de ses sels, en particulier environ 4%, 5%, 10%, 15%, ou plus, notamment au moins 20%.

Dans un mode de réalisation préféré, une composition vétérinaire de l'invention comprend au moins 80% en poids d'isométamidium pur ou d'un de ses sels et au moins 4% en poids du composé disubstitué pur ou d'un de ses sels. Dans un mode plus particulier, l'invention concerne une composition vétérinaire comprenant au moins 80% en poids d'isométamidium pur ou d'un de ses sels et de 4% à 20% en poids du composé disubstitué pur ou d'un de ses sels.

Les compositions selon l'invention peuvent comprendre en outre un ou plusieurs autres actifs. En effet, outre leur teneur définie en actifs purs, les compositions de l'invention peuvent comprendre une quantité contrôlée d'autres actifs, et notamment de l'isomère rouge et/ou de l'isomère bleu et/ou d'autres agents anti-parasitaires.

Ainsi, dans un mode particulier, les compositions de l'invention comprennent en outre de l'isomère rouge ou un de ses sels et/ou de l'isomère bleu ou un de ses sels. La quantité de chacun de ces composés est généralement inférieure à 8% en poids, et préférentiellement inférieure à 5%, 4%, 3%, 2%, voire 1% en poids, par rapport au poids total de la composition. De façon encore plus avantageuse, la composition de l'invention est dépourvue d'isomère rouge) ou de ses sels et/ou est dépourvue d'isomère bleu ou de ses sels.

Une composition vétérinaire particulière de l'invention comprend, par rapport au poids total de la composition :
- au moins 80 % en poids d'isométamidium pur ou un de ses sels,
- au moins 4 % en poids de (3,8-di (3-*m*-amidinophenyltriazeno)-5-éthyl-6-phénylphénanthridium pur ou un de ses sels,
- de 0 à 8 % en poids de 3-(3-*m*-amidinophényl-2-triazeno)-8-amino-5-éthyl-6-phénylphénanthridinium ou un de ses sels, de préférence moins de 5%, 4 %, 3 %, 2 % et de préférence moins de 1 %,
- de 0 à 8 % en poids de (7-(*m*-amidinophenyldiazo)-3,8-diamino-5-ethyl-6-phenylphenanthridinium ou un de ses sels, de préférence moins de 5%, 4 %, 3 %, 2 % et de préférence moins de 1 %, et
- un excipient vétérinairement acceptable.

Une autre composition vétérinaire particulière de l'invention comprend, par rapport au poids total de la composition :
- au moins 70% en poids d'isométamidium pur ou un de ses sels,
- au moins 4% en poids de (3,8-di (3-m-amidinophenyltriazeno)-5-éthyl-6-phénylphénanthridium pur ou un de ses sels,
- moins de 1% en poids de 3-(3-*m*-amidinophényl-2-triazeno)-8-amino-5-éthyl-6-phénylphénanthridinium ou un de ses sels,
- moins de 1% en poids de (7-(*m*-amidinophenyldiazo)-3,8-diamino-5-ethyl-6-phenylphenanthridinium ou un de ses sels, et
- un excipient vétérinairement acceptable.

Les compositions de l'invention peuvent être formulées sous forme d'une solution ou d'une suspension, ou de toute forme appropriée pour une administration parentérale et plus particulièrement pour l'injection intramusculaire ou sous-cutanée. Les compositions sont de préférence solubilisées dans une solution saline.

Les compositions de l'invention comprennent un excipient vétérinairement acceptable, ou en d'autres termes, un excipient acceptable pour un animal non-humain, c'est-à-dire, bien toléré et n'engendrant pas d'effets secondaires, tels que des solvants, des agents de solubilisation, des agents tensioactifs, des antioxydants, des conservateurs, des agents anti-mousse, des complexants, des polymères etc.

Les solvants appropriés comprennent, sans limitation, des solvants aqueux ou des solvants organiques comme un alcool alkylique en C1-C4 tels que l'éthanol, l'isopropanol, le méthanol ; l'acétyltributylcitrate ; les esters d'acides gras tels que par exemple l'ester de diméthyle et l'adipate de diisobutyle ; l'acétone, l'acétonitrile, l'alcool benzylique, le butyldiglycol, le diméthylacétamide, le diméthylformamide, le dipropylène glycol n-butyl éther, l'éthylène glycol monoéthyl éther, l'éthylène glycol éther monométhylique, le diéthylène glycol monoéthyl éther, le monomethylacetamide, le monométhyléther de dipropylèneglycol, les polyoxyéthylène-glycols liquides, le propylèneglycol, les 2-pyrrolidones comme la N-méthyl-pyrrolidone et le N-Octyl-2-pyrrolidone, le propylène carbonate, le diéthylène glycol monoéthyl éther, le dimethyl sulfoxide, l'éthylène glycol, le phtalate de diéthyle, l'éthoxydiglycol, ou leurs combinaisons.

La formulation peut également contenir des agents complexant et des polymères qui assurent une libération contrôlée tels que les huiles, les polyvinylpyrrolidones (PVP), les polyéthylènes glycol, les dextrans, PLA (Acide polylactique), PLGA (Acide polylactique et Glycolique) etc., et leur combinaisons

Les solubilisants comprennent, par exemple, de la polyvinylpyrrolidone. Des exemples d'antioxydants appropriés comprennent l'acide ascorbique, le BHT, le BHA, des esters de l'acide gallique. Les conservateurs appropriés peuvent être par exemple, sans limitation, l'alcool benzylique, le n- butanol, l'acide benzoïque ou de l'acide citrique. Les agents anti -mousse comprennent, sans limitation des supports huileux tel qu'une huile minérale, une huile végétale ou toute autre huile qui est insoluble dans la formation de mousse moyenne, l'huile de silicone, de cire et / ou de la silice hydrophobe, telle que l'éthylène bis stéaramide (EBS), des cires paraffinique, de la silice , de l'alcool gras, savons d'acides gras ou des esters, un composé de silicone, le polyéthylène glycol et les copolymères de polypropylène glycol et les polyacrylates d'alkyle.

Les compositions de l'invention sont typiquement préparées en synthétisant et/ou en purifiant séparément chaque ingrédient puis en les assemblant ou en les ajoutant ou en les mélangeant au sein d'une seule et même composition.

Un objet de l'invention concerne ainsi un procédé de préparation d'une composition vétérinaire de l'invention, comprenant les étapes suivantes :
- préparer séparément l'isométamidium pur et/ou le 3,8-di-(3-m-amidinophenyltriazeno)-5-éthyl-6-phénylphénanthridium pur, ou un de leur sels, et
- assembler lesdits ingrédients, typiquement en présence d'un ou plusieurs excipients acceptables sur le plan vétérinaire.

### Applications

Les compositions vétérinaires de l'invention présentent un effet trypanocide et sont particulièrement adaptées pour un traitement des trypanosomoses et/ou contre un trypanosome, et en particulier dans le traitement curatif et/ou préventif des trypanosomoses chez les mammifères non-humains.

Les trypanosomes sont un genre de l'ordre des Trypanosomatida qui représente un groupe de protistes parasites appartenant aux kinétoplastidés qui sont munis de flagelles locomoteurs. Il en existe une vingtaine d'espèces. A titre d'exemple, on peut citer *Trypanosoma avium, T. brucei, T.brucei brucei, T. gambiese, T. rhodesiense, T. cruzi, T. congolense, T. equinum, T. equiperdum, T. evansi, T. lewisi, T. melophagium, T. percae, T. rangeli, T. rotarium, T. simiae, T. suis, T. theileri, T. triglae, T. vivax*. Ces espèces trypanosomiales sont plus ou moins spécifiques des espèces qu'elles infectent.

Les compositions de l'invention sont efficaces contre différents trypanosomes et présentent un spectre d'activité large. Elles sont efficaces notamment contre les infections par *T. congolense, T. b. brucei, T.brucei, T. evansi* ou *T. vivax*.

Les compositions vétérinaires de l'invention sont utilisables avantageusement pour un traitement curatif et/ou préventif à long terme des trypanosomoses chez les mammifères non-humains, comme par exemple un bovidé, un ovidé, un camélidé, un équidé ou un canidé.

Au sens de l'invention, un traitement « à long terme » signifie un traitement des trypanosomoses ou contre les trypanosomes tels que définis ci-dessus efficace dans la durée, en particulier, une durée supérieure à deux mois, et de préférence supérieure à trois mois.

Les compositions de l'invention sont préférentiellement administrées chez les mammifères non-humains par voie parentérale. Avantageusement, les compositions de l'invention sont administrées sous une forme liquide et injectable, en particulier pour une injection intramusculaire ou sous-cutanée.

Les compositions de l'invention telles que définies ci-dessus présentent préférentiellement une dose d'isométamidium pur ou, alternativement, en association avec le composé disubstitué, allant de 0,05 mg/kg à 5 mg/kg, préférentiellement de 0,05 à 2 mg/kg, et de manière encore plus préférée de 0,1 à 1 mg/kg de poids corporel. Bien entendu, l'homme du métier saura adapter la dose à utiliser en fonction du type de mammifère non-humain à traiter, de sa taille et/ou de son poids.

Un autre objet de l'invention concerne le chlorure d'isométamidium pur à 95 % ou un de ses sels, de préférence le chlorure de chlorhydrate d'isométamidium.

Un objet supplémentaire de l'invention concerne le chlorure de 3,8-di-(3-m-amidinophenyltriazeno)-5-éthyl-6-phénylphénanthridium pur à 95 % ou un de ses sels, de préférence le chlorure de dichlorhydrate de 3,8-di-(3-m-amidinophenyltriazeno)-5-éthyl-6-phénylphénanthridium.

La présente invention concerne également une méthode de traitement curatif et/ou préventif des trypanosomoses chez les mammifères non-humains, caractérisée en ce qu'elle comprend une étape d'administration d'une composition vétérinaire telle que définie ci-dessus.

Un autre objet de l'invention concerne aussi l'utilisation d'une composition vétérinaire telle que définie ci-dessus dans la préparation d'un médicament pour le traitement curatif et/ou préventif des trypanosomoses chez les mammifères non-humains.

D'autres aspects et avantages de l'invention apparaîtront à la lecture des exemples qui suivent, qui doivent être considérés comme illustratifs et non limitatifs.

### EXEMPLES

### Exemple 1 : Activité in vitro de l'isométamidium pur (M&B4180) sur T. congolense et T. b. brucei.

### 1. Matériel et méthodes

### 1.1. Souches de trypanosomes

Les souches *T. congolense* et *T. b. brucei* (formes sanguines) ont été cultivées in vitro selon le protocole décrit par Baltz et al. (Embo. J. 1985, 4, 1273-1277) et Coustou et al. (PLOS Negl. Trop. Dis., 2010, 4, 618).

### 1.2. Composés

L'isométamidium a été synthétisé et a été purifié par HPLC (220 nm). L'analyse de sa structure a été confirmée par LC /MS et par RMN 1H et 13C et un degré de pureté de 99,7 % a été obtenu. L'isométamidium pur ainsi que les formulations commerciales Veridium® et Samorin^{®} ont été dissous dans de l'eau distillée (10 mg / ml) et ont été conservés à -20°C avant utilisation.

### 1.3. Tests in vitro de sensibilité aux composés

Les trypanosomes sont ajoutés dans des plaques de culture cellulaire 96 puits incubées à 34 °C (*T. congolense*) ou à 37 °C (*T. b. brucei*) sous atmosphère de CO₂ (4 %) pendant 4 heures avant l'ajout des composés qui ont été dilués en série 1/10^{eme}. 100 µl de chaque dilution ont été ensuite ajoutés à 100 µl de culture dans les puits (2 puits pour chaque dilution de 1 mg/ml jusqu'à 1.10⁻¹⁹ mg/ml) puis le milieu est incubé à 34 °C (*T. congolense*) ou à 37 °C (*T. b. brucei*) sous atmosphère de CO₂ (4 %) pendant 48 heures. Les IC₅₀ (Concentration qui réduit de 50% la population parasitaire au bout de 48 heures de culture) ont été calculées en utilisant le logiciel de traitement de données Sigmaplot version 12.

### 2. Résultats

Les résultats des IC₅₀ obtenues sont illustrés dans le tableau 1 ci-après.

**Tableau 1 :**

| IC₅₀ (ng/ml) | | |
|---|---|---|
| | *T. congolense* | *T. b. brucei* |
| Isométamidium pur | 0,56 ± 0,05 | 9,24 ± 2,13 |
| Veridium^{®} | 0,82 ± 0,25 | 11,06 ± 3,02 |
| Samorin^{®} | 1,75 ± 0,50 | 11,78 ± 4,88 |

Après 48 heures d'exposition de *T. congolense* avec les différents composés testés, les inventeurs ont démontré que l'isométamidium pur (IC₅₀ = 0,56 ng/ml) est plus actif que le Veridium^{®} (IC₅₀ = 0,82 ng/ml) et le Samorin^{®} (IC₅₀ = 1,75 ng/ml).

Après 48 heures d'exposition de *T. b. brucei* avec les différents composés testés, les inventeurs ont montré également que l'isométamidium pur (IC₅₀ = 9,24 ng/ml) présente l'activité trypanocide la plus importante.

### 3. Conclusions

L'activité trypanocide optimale est obtenue avec l'isométamidium pur. Par conséquent les inventeurs ont démontré qu'une composition d'isométamidium pur a une activité trypanocide accrue observée directement sur le trypanosome par rapport aux formulations commerciales Veridium^{®} et Samorin^{®}.

### Exemple 2 : Activité in vitro de l'isomère rouge (M&B3989), de l'isomère bleu (M&B4250) et du composé disubstitué (M&B4596) sur T. congolense et T. b. brucei.

### 1. Matériel et méthodes

Les souches *T. congolense* et *T. b. brucei* (formes sanguines), les différents composés et leur IC₅₀ ont été, respectivement, cultivées in vitro, préparés et évaluées selon le protocole décrit dans l'exemple 1. Les puretés respectives pour chaque composé obtenu étaient de 97,6 % pour l'isomère bleu, 95,4% pour l'isomère rouge et 97,9 % pour le composé disubstitué.

### 2. Résultats

Les résultats des IC₅₀ sont illustrés dans le tableau 2 ci-dessous.

**Tableau 2 :**

| IC₅₀ (ng/ml) | | |
|---|---|---|
| | *T. congolense* | *T. b. brucei* |
| Isomère rouge | 3,63 ± 0,55 | 202,15 ± 62,92 |
| Isomère bleu | 7,11 ± 0,76 | 12,01 ± 2,22 |
| Composé disubstitué pur | 66,27 ± 14,37 | >1000 |
| Veridium^{®} | 0,82 ± 0,25 | 11,06 ± 3,02 |
| Samorin^{®} | 1,75 ± 0,50 | 11,78 ± 4,88 |

Après 48 heures d'exposition de *T. congolense* avec les différents composés testés, les inventeurs ont observé une activité trypanocide des isomères rouge (IC₅₀ = 3,63 ng/ml) et bleu (IC₅₀ = 7,11 ng/ml) jusqu'à environ 10 fois inférieure à celle observées avec les formulations commerciales Veridium^{®} et Samorin^{®}. Le composé disubstitué (IC₅₀ = 66,27 ng/ml) présente l'activité *in vitro* trypanocide la plus faible.

Après 48 heures d'exposition de *T. b. brucei* avec les différents composés testés, les inventeurs ont montré également que l'isomère bleu (IC₅₀ = 12,01 ng/ml) présente une activité comparable aux activités trypanocide observées avec les formulations Veridium^{®} (IC₅₀ = 11,06 ng/ml) et Samorin^{®} (IC₅₀ = 11,78 ng/ml). Par contre, l'isomère rouge (IC₅₀ = 202,15 ng/ml) présente une activité trypanocide environ 20 fois inférieure à celles observée avec les compositions commerciales. Le composé disubstitué (IC₅₀ > 1000 ng/ml) est inactif sur *T. b. brucei.*

### 3. Conclusions

En comparant les activités trypanocides illustrées dans le tableau 2, les inventeurs ont démontré que les isomères rouges et bleus nuisaient considérablement à l'activité trypanocide des formulations commerciales Veridium^{®} et Samorin^{®}.

Au vu des résultats obtenus dans les exemples 1 et 2, les inventeurs ont démontré qu'une composition enrichie en isométamidium pur au détriment des composés rouges et bleus permet d'obtenir une activité trypanocide améliorée.

### Exemple 3 : Activités curative et préventive in vivo sur T. congolense

### 1. Matériel et méthodes

### 1.1. Souche de trypanosome et composés

La souche *T. congolense* est cultivée et les différents composés sont préparés selon les protocoles de culture et de préparation décrits pour les tests *in vitro* (Exemples 1 et 2).

### 1.2. Tests in vivo

Des parasites de la souche de *T. congolense* ont été initialement propagés dans des souris femelles swiss (CD1) âgées de 8 semaines (200 mg/kg). Ces souris sont ensuite infectés par voie intrapéritonéale avec 10⁵ trypanosomes de souche *T. congolense* dans un volume final de 200 µl de tampon phosphate glucose (137 mM NaCl, 10 mM phosphate, 2,7 nM KCl, pH 7,4, 1% glucose). Les souris sont alors traitées par les composés à tester 24 heures après l'infection (5 souris traités par composé à tester + un groupe témoin). Chaque composé testé est injecté par voie intrapéritonéale et à des concentrations de 1 et 0,1 mg/kg de poids corporel dans un volume final de 100 µl d'eau stérile. Les souris témoins non traitées reçoivent 100 µl d'eau stérile après infection.
La parasitémie a été évaluée 2 fois par semaine au microscope à partir d'échantillons de sang prélevés sur la veine caudale.
Afin d'évaluer la persistance des composés, les souris qui étaient parasite-négatives après un mois ont été ré-infectées avec 10⁵ trypanosomes. La réinfection a été répétée après le deuxième et troisième mois à partir de l'infection initiale et du traitement si les souris infectées étaient toujours aparasitémiques (sans parasites dans le sang). La réinfection a été réalisée avec des parasites provenant de souris témoins infectées de manière chronique pour éviter une réponse immunitaire immédiate due à la présence d'anticorps homologues anti-VSG. Deux souris non-traitées (Témoin) ont été infectées à chaque infection et réinfection. Le traitement a été considéré inefficace quand plus de 2 souris sur 5 dans un groupe étaient parasite-positives.

### 2. Résultats

Les résultats *trypanocides in vivo* des différents composés aux doses de 0,1 et 1 mg/kg sont illustrés dans le tableau 3 ci-après.

**Tableau 3 :**

| | | | Nombre de souris avec une parasitemia positive | | | |
|---|---|---|---|---|---|---|
| Drogue | Dose (mg/kg) | n | Nombre d'infections expérimentales successives | | | |
| | | | 1 mois | 2 mois | 3 mois | 4 mois |
| Samorin^{®} | 1 | 5 | 0 | 0 | 1 | 5 |
| Veridium^{®} | 0.1 | 5 | 0 | 0 | 0^{a} | 4^{a} |
| | 1 | 5 | 0 | 0 | 1 | 5 |
| isometamidium | 0.1 | 5 | 0 | 0 | 0 | 5 |
| | 1 | 5 | 0 | 0 | 0 | 5 |
| Isomère rouge | 0.1 | 5 | 0 | 5 | ND^{b} | ND |
| | 1 | 5 | 0 | 1 | 5 | ND |
| Isomère bleu | 0.1 | 5 | 0 | 5 | ND | ND |
| | 1 | 5 | 0 | 4 | ND | ND |
| Composé disubstitué | 0.1 | 5 | 5 | ND | ND | ND |
| | 1 | 5 | 0 | 0 | 1 | 4 |

| | | | | | | |
|---|---|---|---|---|---|---|
| *^{a} Une souris est morte après la seconde injection changeant le nombre de souris du groupe à 4.* *^{b}ND: Le traitement a été arrêté car plus de 2 souris par groupes étaient infectées.* | | | | | | |

Les résultats obtenus pour une dose de 0,1 mg/kg montrent que tous les composés, excepté le composé disubstitué, présentent une activité trypanocide curative et préventive après un mois de traitement. L'isométamidium pur et le Veridium^{®} dès une dose de 0,1 mg/kg protègent les souris d'une infection pendant trois mois successifs contrairement aux composés rouge et bleu qui ne sont actifs que pendant un mois à cette même dose.

Les résultats obtenus pour une dose de 1 mg/kg de chaque composé montrent que toutes les souris sont parasite-négatives un mois après l'administration. A cette dose et au bout d'un mois, l'ensemble des composés testés, y compris le composé disubstitué, présentent donc tous une activité trypanocide.
L'isométamidium pur et le composé disubstitué sont aussi actifs que les produits Veridium^{®} et Samorin^{®} puisque seule une souris est parasite-positive 3 mois successifs après un traitement avec 1 mg/kg de composé disubstitué. En outre, la seule souris parasite-négative observée après 4 mois successifs a été traitée avec le composé disubstitué.
Par ailleurs, l'isomère bleu est le moins efficace car 4 souris sont parasite-positif après 2 injections.

### 3. Conclusions

Les résultats observés *in vivo* à 0,1 mg/kg et 1 mg/kg confirment l'activité trypanocide améliorée d'une composition enrichie en isométamidium pur au détriment des isomères rouge et bleu.

En outre, des compositions d'isométamidium pur enrichies en composé disubstitué permettent aussi d'obtenir une activité trypanocide à plus long terme (supérieure à 3 mois).

## Revendications

1. Composition vétérinaire comprenant de l'isométamidium pur à au moins 95%, ou un de ses sels, et un excipient vétérinairement acceptable.

2. Composition selon la revendication 1, comprenant au moins 55 % en poids d'isométamidium pur ou d'un de ses sels, par rapport au poids total de la composition.

3. Composition selon la revendication 1 ou 2, comprenant au moins 80 %, de préférence au moins 85 %, plus préférentiellement au moins 90 % d'isométamidium pur ou un de ses sels, par rapport au poids total de la composition.

4. Composition selon l'une quelconque des revendications 1 à 3, comprenant en outre du 3,8-di-(3-*m*-amidinophenyltriazeno)-5-éthyl-6-phénylphénanthridium pur ou un de ses sels.

5. Composition selon la revendication 4, comprenant au moins 4 % en poids de 3,8-di-(3-*m*-amidinophenyltriazeno)-5-éthyl-6-phénylphénanthridium pur ou un de ses sels, par rapport au poids total de la composition.

6. Composition selon l'une quelconque des revendications 1 à 5, **caractérisée en ce qu'**elle comprend moins de 8 % en poids de 3-(3-m-amidinophényl-2-triazeno)-8-amino-5-éthyl-6-phénylphénanthridinium, par rapport au poids total de la composition, de préférence moins de 5%.

7. Composition selon la revendication 6, **caractérisée en ce qu'**elle est dépourvue de 3-(3-m-amidinophényl-2-triazeno)-8-amino-5-éthyl-6-phénylphénanthridinium.

8. Composition selon l'une quelconque des revendications 1 à 7, **caractérisée en ce qu'**elle comprend moins de 8 % en poids de 7-(*m*-amidinophenyldiazo)-3,8-diamino-5-ethyl-6-phenylphenanthridinium, par rapport au poids total de la composition, de préférence moins de 5%.

9. Composition selon la revendication 8, **caractérisée en ce qu'**elle est dépourvue de 7-(m-amidinophenyldiazo)-3,8-diamino-5-ethyl-6-phenylphenanthridinium.

10. Composition selon l'une quelconque des revendications 1 à 9, comprenant, par rapport au poids total de la composition :
- au moins 80 % en poids d'isométamidium pur ou un de ses sels,
- au moins 4 % en poids de 3,8-di-(3-m-amidinophenyltriazeno)-5-éthyl-6-phénylphénanthridium pur ou un de ses sels,
- de 0 à 8 % en poids de 3-(3-m-amidinophényl-2-triazeno)-8-amino-5-éthyl-6-phénylphénanthridinium,
- de 0 à 8 % en poids de 7-(m-amidinophenyldiazo)-3,8-diamino-5-ethyl-6-phenylphenanthridinium, et
- un excipient vétérinairement acceptable.

11. Composition selon l'une quelconque des revendications 1 à 10, **caractérisée en ce que** les sels sont des chlorhydrates ou du chlorure de chlorhydrates.

12. Composition vétérinaire selon l'une quelconque des revendications 1 à 11, pour son utilisation dans le traitement des trypanosomoses et/ou contre un trypanosome chez un mammifère non humain.

13. Composition selon la revendication 12, pour son utilisation dans le traitement curatif et/ou préventif des trypanosomoses chez les mammifères non-humains.

14. Composition selon la revendication 12, pour son utilisation dans le traitement contre un trypanosome choisi parmi *T. congolense, T. b. brucei, T. brucei, T. evansi* et *T. vivax*.

15. Composition pour son utilisation selon l'une quelconque des revendications 12 à 14, **caractérisée en ce que** le mammifère non-humain est un bovidé, un ovidé, un camélidé, un équidé ou un canidé.

16. Composition pour son utilisation selon l'une quelconque des revendications 12 à 15, **caractérisée en ce que** son administration se fait par voie parentérale, de préférence par injection intramusculaire ou sous-cutanée.

17. Procédé de préparation d'une composition vétérinaire telle que définie selon l'une quelconque des revendications 1 à 11, comprenant les étapes suivantes :
- préparer séparément l'isométamidium pur et/ou le 3,8-di-(3-m-amidinophenyltriazeno)-5-éthyl-6-phénylphénanthridium pur, ou un de leur sels, et
- assembler lesdits ingrédients, de préférence en présence d'un excipient vétérinairement acceptable.

18. Chlorure d'isométamidium pur à au moins 95 % ou un de ses sels, de préférence le chlorure de chlorhydrate d'isométamidium.

19. Chlorure de (3,8-di-(3-*m*-amidinophenyltriazeno)-5-éthyl-6-phénylphénanthridium pur à au moins 95 % ou un de ses sels, de préférence le chlorure de dichlorhydrate de (3,8-di-(3-*m*-amidinophenyltriazeno)-5-éthyl-6-phénylphénanthridium.
